# EUROPEAN PATENT APPLICATION

(11) **EP 3 884 938 A1**
(43) Date of publication of application: **29.09.2021**
(21) Application number: 20165735.0
(22) Date of filing: 25.03.2020
(51) Int. Cl.: A61K 31/357, A61P 31/14, A61K 36/282

(54) **1,2,4-TRIOXANE COMPOUNDS AND COMPOSITIONS COMPRISING THE SAME FOR USE IN THE TREATMENT OF COVID-19**

(71) Applicant: ArtemiFlow GmbH, 14476 Potsdam (DE)
(72) Inventor: The designation of the inventor has not yet been filed

(57) **Abstract**

The present invention relates to antiviral 1,2,4-trioxane compounds and compositions comprising the same that are effective inhibitors of severe acute respiratory syndrome coronavirus 2 (SARS-CoV-2) replication, and are thus useful to treat the coronavirus disease 2019 (COVID-19). The invention further provides pharmaceutical compositions containing such antiviral compounds and antiviral compositions, and methods of using these antiviral compounds, antiviral compositions and pharmaceutical compositions in the treatment and prevention of COVID-19.

## Description

### Field of the invention

The present invention relates to antiviral 1,2,4-trioxane compounds and compositions comprising the same that are effective inhibitors of the severe acute respiratory syndrome coronavirus 2 (SARS-CoV-2) replication, and are thus useful to treat the coronavirus disease 2019 (COVID-19). The invention further provides pharmaceutical compositions containing such antiviral compounds and antiviral compositions, and methods of using these antiviral compounds, antiviral compositions and pharmaceutical compositions in the treatment and prevention of COVID-19.

### Background

The severe acute respiratory syndrome coronavirus 2 (SARS-CoV-2) is a positive-sense single-stranded RNA virus that is contagious in humans and is the cause of the coronavirus disease 2019 (COVID-19) that affects all populations, worldwide, including adults and children with normal or compromised immune systems. While often asymptomatic in healthy individuals, SARS-CoV-2 can become life-threatening in immunocompromised individuals. Mortality, depending on average age of population, testing strategy, and health system capacity are estimated to range from 0.5% to 5% of infected individuals.

SARS-CoV-2 is a member of the subgenus Sarbecovirus (beta-CoV lineage B). SARS-CoV-2 is unique among known beta-coronaviruses in its incorporation of a polybasic cleavage site, a characteristic known to increase pathogenicity and transmissibility in other viruses. Currently, no effective treatments for infections are available. Medications commonly used against malaria such as chloroquine, or ebola such as remdesivir, as well as other antiviral drug compounds have been considered for repurposing.

Further, herbal treatments used in Traditional Chinese Medicine have been explored to treat coronavirus infections during the SARS-CoV and MERS-CoV outbreaks.

As earlier disclosed by Li et al., Antiviral Research 67, (2005), p. 18-23 extracts of *Lycoris radiata* containing lycorine are even more effective against SARS-CoV than alcoholic extracts of sweet wormwood (*Artemisia annua*)*.*

The review of Efferth, Biology Advances 36 (2018), 1730-1737 on antiviral activity of artemisinin type compounds draws the conclusion that *Artemisia annua* extracts containing artemisinin and other compounds seem to be active against double stranded DNA viruses but less active towards other viruses and in particular leaves the question open whether such extracts are effective against single stranded RNA viruses.

### Summary of the invention

The present invention relates to antiviral compounds and antiviral compositions that exhibit potent antiviral activity against SARS-CoV-2 in vitro, have a good safety profile and thus provide an opportunity for a broad clinical use. The invention also provides pharmaceutical compositions comprising such antiviral compounds or antiviral compositions as well as methods to use the antiviral compounds, antiviral compositions and pharmaceutical compositions to inhibit, SARS-CoV-2 replication or reactivation, and to treat disease conditions associated with or caused by SARS-CoV-2. Further objects of this invention are described herein below.

In one aspect, the invention relates to antiviral compounds comprising at least one 1,2,4-trioxane moiety for use in the treatment of COVID-19.

### Detailed description

For purposes of interpreting this specification, the following definitions will apply, and whenever appropriate, terms used in the singular will also include the plural.

Terms used in the specification have the following meanings unless the context clearly indicates otherwise:
As used herein, the term "subject" refers to an animal. In certain aspects, the animal is a mammal. A subject also refers to for example, primates (e.g., humans), cows, sheep, goats, horses, dogs, cats, rabbits, rats, mice, fish, birds and the like. In certain embodiments, the subject is a human. A "patient" as used herein refers to a human subject.

As used herein, the term "inhibition" or "inhibiting" refers to the reduction or suppression of a given condition, symptom, or disorder, or disease, or a significant decrease in the baseline activity of a biological activity or process.

As used herein, the term "treating" or "treatment" of any disease or disorder refers in one embodiment, to ameliorating the disease or disorder i.e. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment. "treating" or "treatment" refers to modulating the disease or disorder, either physically, e.g. stabilization of a discernible symptom, physiologically, e.g., stabilization of a physical parameter, or both. In yet another embodiment, "treating" or "treatment" refers to preventing or delaying the onset or development or progression of the disease or disorder.

As used herein, the term "a," "an," "the" and similar terms used in the context of the present invention especially in the context of the claims are to be construed to cover both the singular and plural unless otherwise indicated herein or explicitly contradicted by the context.

All methods described herein can be performed in any suitable order unless otherwise indicated herein or otherwise clearly contradicted by context. The use of any and all examples, or exemplary language e.g. "such as" provided herein is intended merely to better illustrate the invention and does not pose a limitation on the scope of the invention otherwise claimed.

"Optionally substituted" or "substituted" means one or more hydrogen atoms at any position in the molecule or moiety referred to can be substituted by any one or any combination of substituents with their number, placement and selection being understood to encompass only those substitutions that a skilled chemist would expect to be reasonably stable.

Various embodiments of the invention are described herein. It will be recognized that features specified in each embodiment may be combined with other specified features to provide further embodiments.

The antiviral compounds comprising at least one 1,2,4-trioxane moiety are those comprising at least one 1,2,4-trioxane ring which is optionally, but preferably substituted.

In one embodiment the antiviral compounds comprising at least one 1,2,4-trioxane moiety are selected from those of formulae (I) to (V) 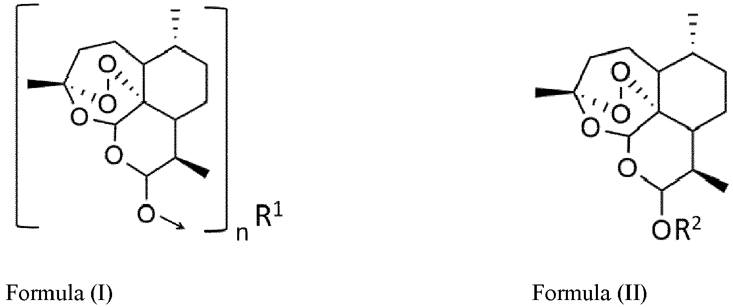 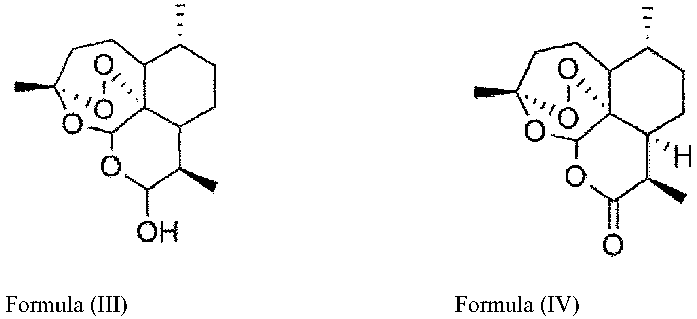 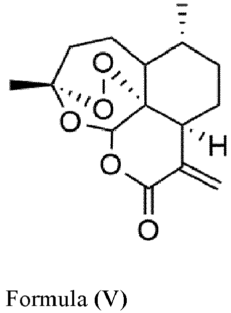 and, where applicable, pharmaceutically acceptable salts of the aforementioned compounds of formulae (I) and (II).

In formula (I)
the arrow denotes the bond between the depicted oxygen atom to the residue R¹
- n: is an integer of more than 1, preferably 2 to 10, more preferably 2, 3 or 4 and even more preferably 2 or 3
- R¹: is a residue that is n times substitued by the residue depicted in the rounded bracket, and is preferably C₁-C₁₈-alkyl or C₂-C₁₈-alkenyl or -(CO)ₙ(R³), wherein the carboyl groups together with the oxygen bound to the residue R¹ form a carboxylic ester moiety and R³ is C₁-C₁₈-alkane-n-yl or C₂-C₁₈-alkene-n-yl
whereby
the aforementioned C₁-C₁₈-alkyl, C₂-C₁₈-alkenyl, C₁-C₁₈-alkane-n-yl, C₂-C₁₈-alkene-n-yl groups are
- either not, once, twice, or more than twice interrupted by non-successive functional groups selected from the group consisting of:
   -O-, -S-, -SO₂-, -SO-, -SO₂NR⁴-, NR⁴SO₂-, -NR⁴-, -CO-, -O(CO)-, (CO)O-, -O(CO)O-, -NR⁴(CO)NR⁴-, NR⁴(CO)-, -(CO)NR⁴-, -NR⁴(CO)O-, -O(CO)NR⁴-,
   and
- either not, additionally, or alternatively either once, twice or more than twice interrupted by bivalent residues selected from the group consisting of heterocyclo-diyl, and aryldiyl,
   and
- either not, additionally, or alternatively either once, twice or more than twice substituted by substituents selected from the group consisting of:
   hydroxy, halogen, cyano, azido, C₆-C₁₄-aryl, C₁-C₈-alkoxy, C₁-C₈-alkylthio, -SO₃M,-COOM, PO₃M₂, -PO(N(R⁵)₂)₂, PO(OR⁵)₂, -SO₂N(R⁴)₂, -N(R⁴)₂, -CO₂N(R⁵)₂, -COR⁴,-OCOR4, -NR⁴(CO)R⁵, -(CO)OR⁴, -NR⁴(CO)N(R⁴)₂

In formula (II)
- R²: is C₁-C₁₈-alkyl or C₂-C₁₈-alkenyl or -(CO)R³, wherein the carboyl groups together with the oxygen bound to the residue R¹ form a carboxylic ester moiety and R³ is C₁-C₁₈-alkyl or C₂-C₁₈-alkenyl whereby
the aforementioned C₁-C₁₈-alkyl and C₂-C₁₈-alkenyl groups are
- either not, once, twice, or more than twice interrupted by non-successive functional groups selected from the group consisting of:
   -O-, -S-, -SO₂-, -SO-, -SO₂NR⁴-, NR⁴SO₂-, -NR⁴-, -CO-, -O(CO)-, (CO)O-,-O(CO)O-, -NR⁴(CO)NR⁴-, NR⁴(CO)-, -(CO)NR⁴-, -NR⁴(CO)O- or -O(CO)NR⁴-
   and
- either not, additionally, or alternatively either once, twice or more than twice interrupted by bivalent residues selected from the group consisting of heterocyclo-diyl, and aryldiyl,
   and
- either not, additionally, or alternatively either once, twice or more than twice substituted by substituents selected from the group consisting of:
   hydroxy, halogen, cyano, azido, C₆-C₁₄-aryl, C₁-C₈-alkoxy, C₁-C₈-alkylthio,-SO₃M, -COOM, PO₃M₂, -PO(N(R⁵)₂)₂, PO(OR⁵)₂, -SO₂N(R⁴)₂, -N(R⁴)₂,-CO₂N(R⁵)₂, -COR⁴, -OCOR⁴, -NR⁴(CO)R⁵, -(CO)OR⁴ or -NR⁴(CO)N(R⁴)₂
whereby in all formulae above where used
- R⁴: is independently selected from the group consisting of hydrogen, C₁-C₈-alkyl, C₆-C₁₄-aryl, and heterocyclyl or N(R⁴)₂ as a whole is a N-containing heterocycle,
- R⁵: is independently selected from the group consisting of C₁-C₈-alkyl, C₆-C₁₄-aryl, and heterocyclyl or N(R⁵)₂ as a whole is a N-containing heterocycle and
- M: is hydrogen, or 1/q equivalent of an q-valent metal ion or is an ammonium ion or a guanidinium ion or a primary, secondary, tertiary or quarternary organic ammonium ion, in particular those of formula [N(C₁-C₁₈-alkyl)ₛHₜ]⁺ wherein s is 1,2,3 or 4 and t is (4-s).

As used herein, and unless specifically stated otherwise, C₁-C₁₈-alkyl, C₁-C₁₈-alkene-n-yl, C₁-Cs-alkyl, C₁-C₈-alkoxy and C₁-C₈-alkylthio include straight-chained or, for C₃-C₁₈ or C₃-C₈ also cyclic either in part or as a whole, branched or unbranched alkyl, alkoxy, and alkylthio substituents having the given number of carbon atoms in the substituent as such.

As used herein, and unless specifically stated otherwise, C₂-C₁₈-alkenyl include straight-chained or, for C₅-C₁₈ also cyclic either in part or as a whole, branched or unbranched alkenyl, having the given number of carbon atoms in the substituent as such.

As used herein, and unless specifically stated otherwise, C₆-C₁₄-aryl, C₆-C₁₄-aryloxy, and C₆-C₁₄-arylthio denote carbocyclic aromatic substituents having six to fourteen carbon atoms within the aromatic system as such, i.e. without carbon atoms of substituents, preferably phenyl (C₆), naphthyl (C₁₀), phenanthrenyl and anthracenyl (each C₁₄), whereby said carbocyclic, aromatic substituents are either unsubstituted or substituted by up to five identical or different substituents per cycle. For example and with preference, the substituents are selected from the group consisting of fluoro, chloro, C₁-C₁₈-alkyl, C₁-C₁₈-alkoxy, C₆-C₁₄-aryl.

In a more preferred embodiment the carbocyclic, aromatic substituents are unsubstituted.

Specific examples of C₁-C₁₈-alkyl are methyl, ethyl, n-propyl, isopropyl, n-butyl, sec-butyl, tert-butyl, n-pentyl, isopentyl, tert.-pentyl, neopentyl, cyclohexyl, n-hexyl, n-heptyl, n-octyl and isooctyl, n-decyl, n-dodecyl n-hexadecyl, n-octadecyl.

Specific examples of C₁-C₈-alkoxy-substituents are methoxy, ethoxy, isopropoxy, n-propoxy, n-butoxy, sec.-butoxy, tert-butoxy and cyclohexyloxy.

Specific examples of C₁-C₈-alkylthio-substituents are methylthio and ethylthio.

Specific examples of C₆-C₁₄-aryl are phenyl, o-, m-and p-tolyl.

A further specific example of an C₆-C₁₄-aryl -substituent is phenoxy.

A further specific example of an C₆-C₁₄-aryl -substituent is phenylthio.

Preferred compounds of formula (II) are those of formula (IIa), artemether, and of formula (IIb), artesunate, and pharmaceutically acceptable salts of artesunate.

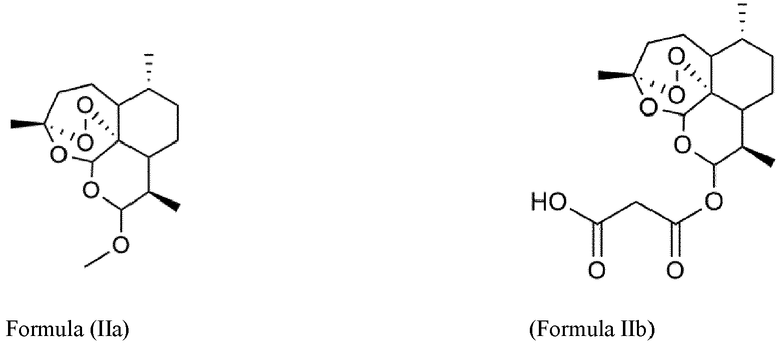

The compound of formula (III) is dihydroartemisin.

The compound of formula (IV) is artemisinin.

The compound of formula (V) is artemisitene.

In one embodiment of the invention antiviral compositions are used which contain more than one antiviral compound comprising at least one 1,2,4-trioxane moiety and preferably more than one antiviral compound selected from those of formulae (I) to (V) above and, where applicable, pharmaceutically acceptable salts of such compounds.

In one preferred embodiment the antiviral composition comprises at least two, for example two, three, four or all the compounds selected from formula (IIa), (IIb), (III), (IV) and (V).

In another embodiment the antiviral compositions further include at least one compound, for example one, two, three, four, five, six, seven, eight, nine, ten or all compounds selected from the group consisting of those of formulae (VI) to (XVI)

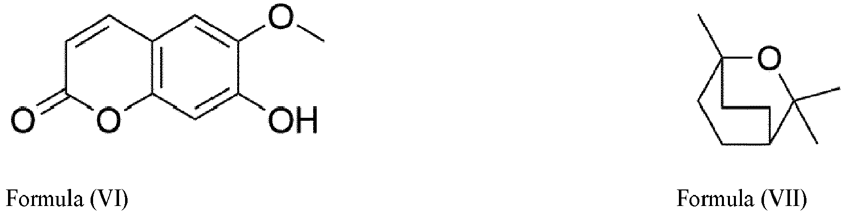

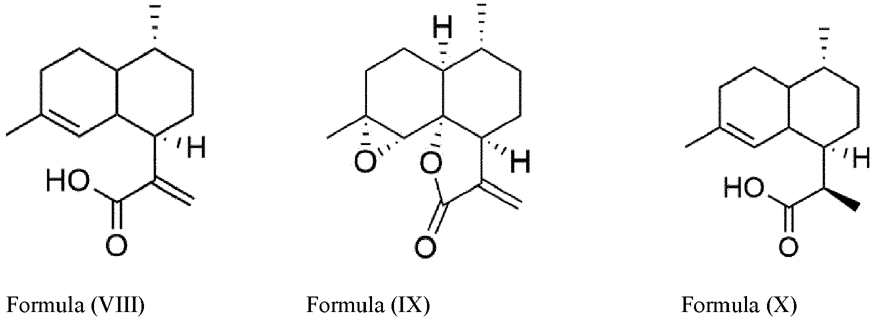

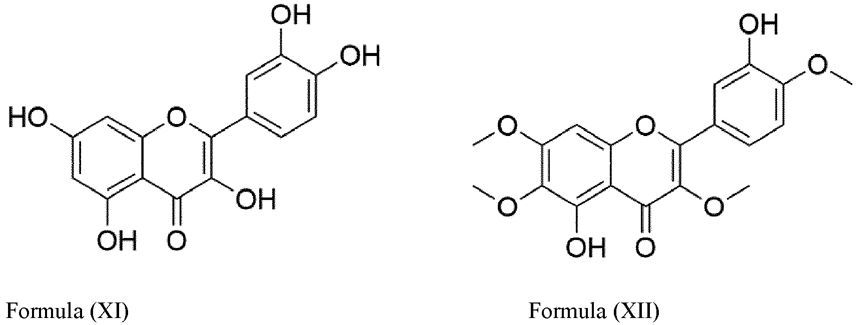

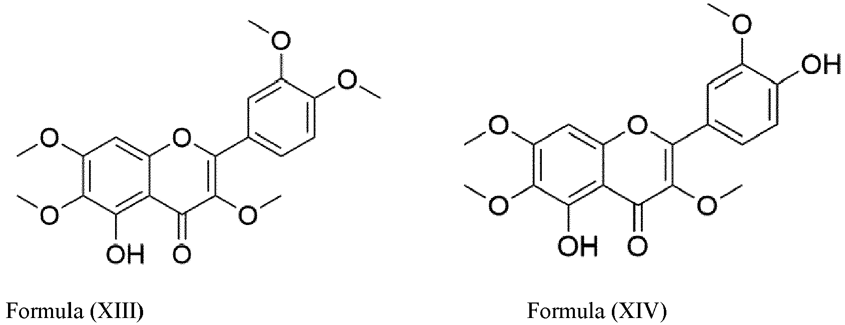

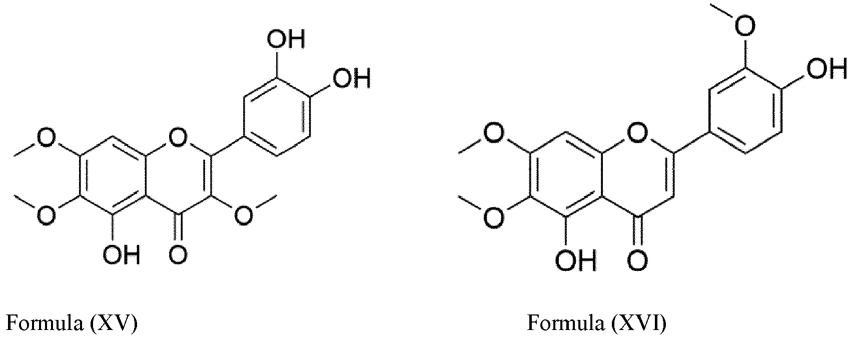

The compound of formula (VI) is scopoletin.

The compound of formula (VII) is 1,8-cineole.

The compound of formula (VIII) is artemisinic acid.

The compound of formula (IX) is arteannuin-B.

The compound of formula (X) is dihydroartemisinic acid.

The compound of formula (XI) is fisetin.

The compound of formula (XII) is casticin.

The compound of formula (XII) is artemetin.

The compound of formula (XIV) is chrysoplenetin.

The compound of formula (XV) is chrysoplenol-D.

The compound of formula (XVI) is cirsilineol.

The invention further comprises a pharmaceutical composition, comprising an antiviral compound or antiviral composition of any of the preceding embodiments.

According to a further aspect of this invention the pharmaceutical composition further comprises a therapeutically effective amount of at least one antiviral agent other than the antiviral compounds comprising at least one 1,2,4-trioxane moiety mentioned above.

The invention also provides the use of the antiviral compounds, antiviral compositions, and a pharmaceutical compositions as described hereinabove for the treatment of a SARS-CoV-2 infection.

The invention further encompasses the use of the antiviral compounds, antiviral compositions, and a pharmaceutical compositions as described hereinabove for the treatment of COVID-19 in a human being having or at risk of having the disease.

Another aspect of the invention encompasses a method of treating a disease or disorder in a patient which may be induced, exacerbated, and/or accelerated by a SARS-CoV-2 infection or COVID-19 by administering to the human being an antivirally effective amount of a composition according to the invention alone or in combination with at least one other antiviral agent, administered together or separately.

Another aspect of the invention relateds to a method of treating or preventing COVID-19 and/or a SARS-CoV-2 infection in a human being by administering to the human being an antivirally effective amount of an antiviral compound or antiviral composition or pharmaceutical composition according to the invention alone or in combination with at least one other antiviral agent, administered together or separately.

An additional aspect of this invention encompasses an article of manufacture comprising a pharmaceutical composition as described hereinabove to treat COVID-19 and/or a SARS-CoV-2 infection; and packaging material comprising a label which indicates that the composition can be used to treat COVID-19 and/or infection by SARS-CoV-2.

Yet another aspect of this invention relates to a method of inhibiting the replication of SARS-CoV-2 comprising exposing the virus to an effective amount of the antiviral compound or antiviral composition or pharmaceutical compositions decribed above under conditions where replication of SARS-CoV-2 is inhibited. This method can be practiced in vitro or in vivo.

The scope of the invention further includes the use of such antiviral compound or antiviral composition or pharmaceutical compositions to inhibit the replication of SARS-CoV-2.

In one embodiment of the invention, the antiviral compounds or antiviral compositions of the invention are is co-administered with at least one additional agent selected from: a virus entry inhibitor, a virus early transcription event inhibitor, another virus RNA polymerase inhibitor such as remdesivir, a virus protease inhibitor such as lopinavir or ritonavir, a virus terminase inhibitor, a virus maturation inhibitor, an inhibitor of another target in the virus life cycle such as , hydroxychloroqione or pharmaceutically acceptable salts thereof and a vaccine.

These additional agents may be combined with the antiviral compound or antiviral composition or pharmaceutical compositions of this invention to create a single pharmaceutical dosage form. Alternatively these additional agents may be separately administered to the patient as part of a multiple dosage form, for example, using a kit. Such additional agents may be administered to the patient prior to, concurrently with, or following the administration of a pharamceutical composition of the invention.

The dose range of the antiviral compounds or antiviral compositions of the invention applicable per day is for example from 0.01 to 100 mg/kg of body weight, preferably from 0.1 to 50 mg/kg of body weight, and even more preferably from 0.5 to 50 mg/kg of body weight.

Each dosage unit may contain from 5% to 95 wt-% of the antiviral compounds or antiviral compositions. Preferably the pharmaceutical compositions according to the invention contain from 20% to 80 wt-% of the antiviral compounds or antiviral compositions.

The actual pharmaceutically effective amount or therapeutic dosage will of course depend on factors known by those skilled in the art such as age and weight of the patient, route of administration, and severity of disease. In any case the combination will be administered at dosages and in a manner which allows a pharmaceutically effective amount to be delivered based upon patient's individual condition.

When the pharmaceutical composition of this invention comprises a combination of an antiviral compound or antiviral composition of the invention and one or more additional therapeutic or prophylactic agent, both the antiviral compound or antiviral composition and the additional agent should be present at dosage levels of between about 10 to 100%, and more preferably between about 10 and 80% of the dosage normally administered in a monotherapy regimen.

Antiviral agents contemplated for use in such combination therapy include agents i.e. compounds or biologicals that are effective to inhibit the formation and/or replication of a virus in a human being, including but not limited to agents that interfere with either host or viral mechanisms necessary for the formation and/or replication of a virus in a human being. Such agents can be selected from: a virus entry inhibitor; a virus early transcription event inhibitor; a virus RNA polymerase inhibitor such as Remdesivir, a virus protease inhibitor such as lopinavir or ritonavir, a virus terminase inhibitor, a virus maturation inhibitor, an inhibitor of another target in the virus life cycle such as chloroquine, hydroxychloroqione, triazavirine or a pharmaceutically acceptable salts of the aformenetioned and a vaccine.

Naturally occuring antiviral compounds and antiviral compositions of the invention may be employed for the purposes of this invention by using the plant *Artemisia Annua* or parts thereof as such or may be obtained via known extraction methods from *Artemisia Annua* as and, where desired, standard workup methods e.g. as published in Triemer et al., Angewandte Chemie, International Edition 57, (2018), p. 5525-5528.

Where e.g. single compounds of the invention are desired to be used extracts of *Artemisia Annua* can be separated in a manner known per se to obtain the individual antiviral compounds for example, by partitioning between polyphasic solvent mixtures, recrystallization and/or chromatographic separation, for example over silica gel or by, e.g., medium pressure liquid chromatography over a reversed phase column or by fractional crystallization.

In one embodiment the *Artemisia Annua* plant is of the Apollon variety, see X. Simmonet et al., "Apollon, a new Artemisia annua variety with high artemisinin content", Planta Medica, 2011, 77(12) which is commercially available from the company Mediplant, Conthey Switzeland

The individual antiviral compounds can be worked up and/or purified according to standard methods, e.g., using chromatographic methods, distribution methods, (re-) crystallization, and the like.

In particular compounds of formulae (IV) to (XVI) were reported to be present in *Artemisia Annua* extracts, see inter alia Czechowski et al., Frontiers in Plant Science, 2019, Vol. 10, Article 984; Zarelli et al., Phytochemical Analysis 2019, 30, 564-571

Synthetic or semi-synthetic antiviral compounds and antiviral compositions of the invention are for example prepared by preparation methods known to those skilled in the art and some of which are published e.g. in Reiter, C., Fröhlich, T., Gruber, L., Hutterer, C., Marschall, M., Voigtländer, C., Friedrich, O., Kappes, B., Efferth, T., Tsogoeva, S.B., 2015a. Highly potent artemisinin-derived dimers and trimers: Synthesis and evaluation of their antimalarial, antileukemia and antiviral activities. Bioorg. Med. Chem. 23 (17), 5452-5458; Reiter, C., Fröhlich, T., Zeino, M., Marschall, M., Bahsi, H., Leidenberger, M., Friedrich, O., Kappes, B., Hampel, F., Efferth, T., Tsogoeva, S.B., 2015b. New efficient artemisinin derived agents against human leukemia cells, human cytomegalovirus and Plasmodium falciparum: 2nd generation 1,2,4-trioxane-ferrocene hybrids. Eur. J. Med. Chem. 97, 164-172; Posner, G.H., Ploypradith, P., Parker, M.H., O'Dowd, H., Woo, S.H., Northrop, J., Krasavin, M., Dolan, P., Kensler, T.W., Xie, S., Shapiro, T.A., 1999. Antimalarial, antiproliferative, and antitumor activities of artemisinin-derived, chemically robust, trioxane dimers. J. Med. Chem. 42 (21), 4275-4280; Paik, I.H., Xie, S., Shapiro, T.A., Labonte, T., Narducci Sarjeant, A.A., Baege, A.C., Posner, G.H., 2006. Second generation, orally active, antimalarial, artemisinin-derived trioxane dimers with high stability, efficacy, and anticancer activity. J. Med. Chem. 49 (9), 2731-2734, Li, Y., Zhu, Y.M., Jiang, H.J., Pan, J.P., Wu, G.S., Wu, J.M., Shi, Y.L., Yang, J.D., Wu, B.A., 2000. Synthesis and antimalarial activity of artemisinin derivatives containing an amino group. J. Med. Chem. 43 (8), 1635-1640; Ren, Y., Yu, J., Kinghorn, A.D., 2016. Development of anticancer agents from plant-derived sesquiterpene lactones. Curr. Med. Chem. 23 (23), 2397-2420. O'Neill, P.M., Searle, N.L., Kan, K.W., Storr, R.C., Maggs, J.L., Ward, S.A., Raynes, K., Park, B.K., 1999. Novel, potent, semisynthetic antimalarial carba analogues of the first-generation 1,2,4-trioxane artemether. J. Med. Chem. 42 (26), 5487-5493 which are hereby incorporated by reference.

Where *Artemisia Annua* is extracted, this may occur using the whole plant or parts thereof such as leaves or stems, whether dried or freshly harvested. Suitable solvents for extraction include hexanes, cyclohexane, supercritical carbon dioxide, hydrofluorocarbon HFC-134a, ionic liquids, water, methanol, ethanol, 1-butanol, acetone, cyclohexanone, toluene, ethyl acetate, acetonitrile, tetrahydrofuran, or mixtures thereof.

Suitable extracts encompass also teas and coffees comprising *Artemisia Annua*. Furthermore, the antiviral compounds of the present invention, including their salts, can also be obtained in the form of their hydrates, or include other solvents used for their crystallization and/or extraction. The compounds of the present invention may inherently or by design form solvates with pharmaceutically acceptable solvents including water; therefore, it is intended that the invention embrace both solvated and unsolvated forms. The term "solvate" refers to a molecular complex of a compound of the present invention including pharmaceutically acceptable salts thereof with one or more solvent molecules. Such solvent molecules are those commonly used in the pharmaceutical art, which are known to be innocuous to the recipient, e.g., water, ethanol, and the like. The term "hydrate" refers to the complex where the solvent molecule is water.

The antiviral compounds of the present invention, including salts, hydrates, and solvates thereof, may inherently or by design form polymorphs. All such polymorphs are encompassed by this invention.

As used herein, the terms "salt" or "salts" refers to an acid addition or base addition salt of a compound of the present invention. "Salts" include in particular "pharmaceutically acceptable salts". The term "pharmaceutically acceptable salts" refers to salts that retain the biological effectiveness and properties of the compounds of this invention and, which typically are not biologically or otherwise undesirable. In some cases, the antiviral compounds of the present invention are capable of forming acid and/or base salts by virtue of the presence of amino and/or carboxyl groups like for artesunate or groups similar thereto.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

Inorganic bases from which salts can be derived include, for example, ammonium salts and metal cations from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The pharmaceutically acceptable salts of the present invention can be synthesized from a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable.

Lists of additional suitable salts can be found, e.g., in "Handbook of Pharmaceutical Salts: Properties, Selection, and Use" by Stahl and Wermuth (Wiley-VCH, Weinheim, Germany, 2002).

Any formula given herein is intended to represent unlabeled forms as well as isotopically labeled forms of the antiviral compounds of the present invention having up to three atoms with non-natural isotope distributions, e.g., sites that are enriched in deuterium or 13C or 15N.

Antiviral compounds of the present invention that contain groups capable of acting as donors and/or acceptors for hydrogen bonds may be capable of forming co-crystals with suitable co-crystal formers. These co-crystals may be prepared from compounds of the present invention by known co-crystal forming procedures. Such procedures include grinding, heating, co-subliming, co-melting, or contacting in solution compounds of the present invention with the co-crystal former under crystallization conditions and isolating co-crystals thereby formed. Suitable co-crystal formers include those described in WO 2004/078163. Hence the invention further provides co-crystals comprising a compound of the present invention.

The pharmaceutical compositions of the invention can be administered by known methods, including oral, parenteral, inhalation, and the like. In certain embodiments, the compound of the invention is administered orally, as a pill, lozenge, troche, capsule, solution or extract such as a tea or other type of infusion beverage, in particular extracts of *Artemisia Annua,* or suspension.

In other embodiments, pharmaceutical compositions of the invention are administered by injection or infusion. Infusion is typically performed intravenously, often over a period of time between about 15 minutes and 4 hours. In other embodiments, pharmaceutical compositions of the invention are administered intranasally or by inhalation; inhalation methods are particularly useful. Pharmaceutical compositions of the invention the present invention exhibit oral bioavailability, so oral administration is sometimes preferred.

The antiviral compounds or antiviral compositions of the present invention may also be used in combination with other agents (combination partners), e.g., an additional antiviral agent other than the antiviral compounds or antiviral compositions of the invention for treatment of a viral infection in a subject.

By the term "combination", is meant either a fixed combination in one dosage unit form, as separate dosage forms suitable for use together either simultaneously or sequentially, or as a kit of parts for the combined administration where a compound of the present invention and a combination partner may be administered independently at the same time or separately within time intervals that especially allow that the combination partners show a cooperative, e.g., synergistic, effect, or any combination thereof.

In certain embodiments of the present invention, the antiviral compounds or antiviral compositions of the present invention are used in combination with at least one further antiviral agent, e.g. a second or third such as those named herein.

The further antiviral agent may be administered in combination with the pharmaceutical compositions of the present inventions wherein the second antiviral agent is administered prior to, simultaneously, or after the compound or compounds of the present invention. When simultaneous administration of a compound of the invention with a further agent is desired and the route of administration is the same, then a compound of the invention may be formulated with such further agent into the same dosage form. An example of a dosage form containing an antiviral compound or antiviral composition of the invention and a further agent is a tablet or a capsule or an inhalable liquid.

In some embodiments, a combination of a antiviral compound or antiviral composition of the invention and a further antiviral agent may provide synergistic activity. The antiviral compound or antiviral composition of the invention and further antiviral agent may be administered together, separate but simultaneously, or sequentially.

An "effective amount" of a compound is that amount necessary or sufficient to treat or prevent an infection with SARS-CoV-2 and/or COVID-19. In an example, an effective amount of a antiviral compound or antiviral composition of the invention is an amount sufficient to treat viral infection with SARS-CoV-2 in a subject.

The effective amount can vary depending on such factors as the size and weight of the subject, the type of illness, or the particular compound of the invention. For example, the choice of the compound of the invention can affect what constitutes an "effective amount." One of ordinary skill in the art would be able to study the factors contained herein and make the determination regarding the effective amount of the compounds of the invention without undue experimentation.

The regimen of administration can affect what constitutes an effective amount. The compound of the invention can be administered to the subject either prior to or after the onset of a viral infection. Further, several divided dosages, as well as staggered dosages, can be administered daily or sequentially, or the dose can be continuously infused, or can be a bolus injection. Further, the dosages of the compound(s) of the invention can be proportionally increased or decreased as indicated by the exigencies of the therapeutic or prophylactic situation.

The antiviral compounds and antiviral compositions of the invention may be used in the treatment of states, disorders, or diseases as described herein, or for the manufacture of pharmaceutical compositions for use in the treatment of these diseases. The invention provides methods of use of compounds of the present invention in the treatment of these diseases or for preparation of pharmaceutical compositions having compounds of the present invention for the treatment of these diseases.

The language "pharmaceutical composition" includes preparations suitable for administration to mammals, e.g., humans. When the antiviral compounds and antiviral compositions of the present invention are administered as pharmaceuticals to mammals, e.g., humans, they can be given per se or as a pharmaceutical composition containing, for example, 0.1 to 99.5% (more preferably, 0.5 to 90%) of at least one antiviral compound or a antiviral composition of the invention or any subgenus thereof as active ingredient in combination with a pharmaceutically acceptable carrier, or optionally two or more pharmaceutically acceptable carriers.

The phrase "pharmaceutically acceptable carrier" is art recognized and includes a pharmaceutically acceptable material, composition or vehicle, suitable for administering the antiviral compounds or a antiviral compositions of the present invention to mammals. The carriers include liquid or solid filler, diluent, excipient, solvent, or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose, and sucrose; starches, such as corn starch and potato starch; cellulose, and its derivatives, such as sodium carboxymethyl cellulose, ethyl cellulose, and cellulose acetate; powdered tragacanth; malt; gelatin; talc; excipients, such as cocoa butter and suppository waxes; oils, such as peanut oil, cottonseed oil, safflower oil, sesame oil, olive oil, corn oil, and soybean oil; glycols, such as propylene glycol; polyols, such as glycerin, sorbitol, mannitol, and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations. Typically, pharmaceutically acceptable carriers are sterilized and/or substantially pyrogen-free.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, α-tocopherol, and the like; and metal chelating agents, such as citric acid, ethylenediamine tetraacetic acid (EDTA), sorbitol, tartaric acid, phosphoric acid, and the like.

Formulations of the present invention include those suitable for oral, nasal, inhalation, topical, transdermal, buccal, sublingual, rectal, vaginal, and/or parenteral administration. The formulations may conveniently be presented in unit dosage form and may be prepared by any methods well known in the art of pharmacy. The amount of active ingredient that can be combined with a carrier material to produce a single dosage form will generally be that amount of the antiviral compound or antiviral composition that produces a therapeutic effect. Generally, out of one hundred per cent, this amount will range from about 1 per cent to about ninety-nine percent of active ingredient, preferably from about 5 per cent to about 80 per cent.

Methods of preparing these formulations or compositions include the step of bringing into association a compound of the present invention with the carrier and, optionally, one or more accessory ingredients. In general, the formulations are prepared by uniformly and intimately bringing into association an antiviral compound or an antiviral composition of the present invention with liquid carriers, or finely divided solid carriers, or both, and then, if necessary, shaping the product.

Formulations of the invention suitable for oral administration may be in the form of capsules, cachets, pills, tablets, lozenges (using a flavored base, for example, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary, or paste.

In solid dosage forms of the invention for oral administration such as capsules, tablets, pills, dragees, powders, granules and the like, the antiviral compounds or antiviral compositions are mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, teas, coffees, suspensions, syrups and elixirs. In addition to the active ingredient, the liquid dosage forms may contain inert diluent commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants that may be required.

Pharmaceutical compositions of this invention suitable for parenteral administration may comprise one or more compounds of the invention in combination with one or more pharmaceutically acceptable carriers such as sterile isotonic aqueous or nonaqueous such as ethanolic solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

The preparations of the present invention may be given orally, pulonary, parenterally, topically, or rectally.

They are of course given by forms suitable for each administration route. For example, they are administered in tablets or capsule form, by injection, inhalation, eye lotion, ointment, suppository, etc., administration by injection, infusion or inhalation; topical by lotion or ointment; and rectal by suppositories.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

Intravenous infusion is sometimes a preferred method of delivery for compounds of the invention. Infusion may be used to deliver a single daily dose or multiple doses. In some embodiments, a compound of the invention is administered by infusion over an interval between 15 minutes and 4 hours, typically between 0.5 and 3 hours. Such infusion may be used once per day, twice per day, or up to three times per day.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, pulmonary, nasally, as by, for example, a spray, rectally, intravaginally, parenterally, intracisternally and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound that is the lowest dose effective to produce a therapeutic effect. Such an effective dose will generally depend upon the factors described above. An effective amount is that amount which prevents or treats SARS-CoV-2 infection.

If desired, the effective daily dose of the active compound may be administered as a single dose per day, or as two, three, four, five, six, or more sub-doses administered separately at appropriate intervals throughout the day, optionally, in unit dosage forms. Compounds delivered orally or by inhalation, are commonly administered in one to four doses per day. Compounds delivered by injection are typically administered once per day, or once every other day. Compounds delivered by infusion are typically administered in one to three doses per day. When multiple doses are administered within a day, the doses may be administered at intervals of about 4 hours, about 6 hours, about 8 hours, or about 12 hours.

While it is possible for a compound of the present invention to be administered alone, it is preferable to administer the compound as a pharmaceutical composition such as those described herein. Thus, methods of using the compounds of the invention include administering the compound as a pharmaceutical composition, wherein at least one compound of the invention is admixed with a pharmaceutically acceptable carrier prior to administration.

The invention further encompasses the use of Compounds of the Invention in combination with immunomodulators.

The compounds and compositions described herein can be used or administered in combination with one or more therapeutic agents that act as immunomodulators, e.g., an activator of a co-stimulatory molecule, or an inhibitor of an immune-inhibitory molecule, or a vaccine.

By "in combination with," it is not intended to imply that the therapy or the therapeutic agents must be administered at the same time and/or formulated for delivery together, although these methods of delivery are within the scope described herein. The immunomodulator can be administered concurrently with, prior to, or subsequent to, one or more compounds of the invention, and optionally one or more additional therapies or therapeutic agents. The therapeutic agents in the combination can be administered in any order. In general, each agent will be administered at a dose and/or on a time schedule determined for that agent. It will further be appreciated that the therapeutic agents utilized in this combination may be administered together in a single composition or administered separately in different compositions. In general, it is expected that each of the therapeutic agents utilized in combination be utilized at levels that do not exceed the levels at which they are utilized individually.

The invention is further exemplified hereinbelow without, however, limiting the scope of the invention.

### Example:

Artesunate is dissolved in dimethyl sulphoxide at 6 different concentrations (0.05, 0.26, 1.3, 6.5, 11.7, 16.9 µM) that are distributed into 96-well sample plates and stored at -80 °C until screening.

The SARS-CoV-2 is propagated in Vero E6 cells at 37 °C in a humidified atmosphere consisting of 5% CO₂. Vero E6 cells are cultured in medium containing 5% heat-inactivated fetal bovine serum (FBS) as well as sodium bicarbonate (3.7 g/L), glucose (4.5 g/L), and 15 mM HEPES buffer. The virus-induced cytopathic effect (CPE) is determined by the MTS method and by visualization of the cellular morphology change. The number of viable cells is correlated with absorbance at 490 nm in MTS assay.

Approximately, 4×10³ Vero E6 cells/well are seeded onto 96-well tissue culture plates with a final volume of 100 µL. They are then cultured for 24 h. Ten µL of artesunate solution (100 µg/ml, 0.26 µM) is added into each well in duplicates before inoculating with the virus stock. The positive control used was Interferon alpha. The viral titers were assessed by cytopathic effect (CPE), determined visually under the light-phase microscope 2-4 days post-infection (PI). The concentration to achieve 90% of cell lysis is used in the artesunate screening. The infected cells with or without compound are incubated at 37 °C in the humidified (5% CO₂) atmosphere for 72 h. Then, 20 µL of MTS/phenazine methosulfate (PMS) is added in each well. The cells are then incubated for another two hours at 37 °C. Finally, 50 µL of 10% SDS is added to stop the color reaction. The plates are then measured at 490 nm using a microplate reader.

The cell morphology changes caused by CPE and the inhibition effects of the compounds prior to the MTS assay are recorded. The dose dependency of artesunate is determined by serial dilutions. The percentage of CPE reduction is calculated by subtracting the mean value of virus-infected cell control (0%) from the measured absorbance. The resulting number is then divided by the measured absorbance of the uninfected cell control (100%). The mean values and the standard deviation are used for data analysis. The EC₅₀ values are determined as the concentration of the compounds needed to achieve the inhibition of SARS-CoV-induced CPE to 50% of the control value (cells without viral infection).

Dried Artemisia annuua plants that were grown using the Apollon variety seeds (Mediplant, Switzerland) in Kentucky, USA are extracted using either hexanes, toluene or ethanol in batch for six hours. The extracts are filtered and lyophilized and thenredissolved in dimethylsulfoxide (DMSO) and stored in 96 well sample plates at -80°C for assays and screening, which is done as described above.

EC₅₀ is in all examples i.e. whether using artesunate or plant extractsasufficiently low to indicate the potential of antiviral compounds and antiviral compositions of the invention against SARS-CoV-2.

## Claims

1. Antiviral compounds comprising at least one 1,2,4-trioxane moiety for use in the treatment of COVID-19.

2. The compounds for use in the treatment of COVID-19 according to claim 1 being selected from
those of formulae (I) to (V) 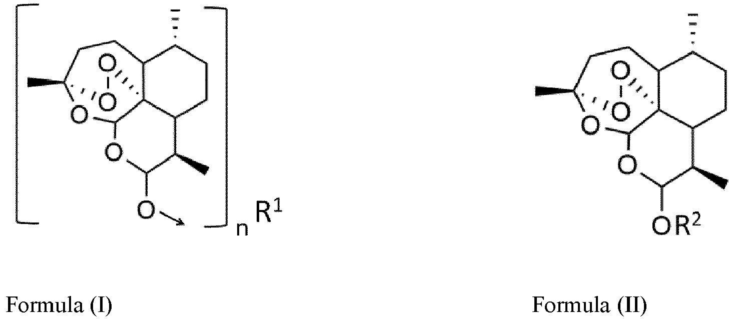 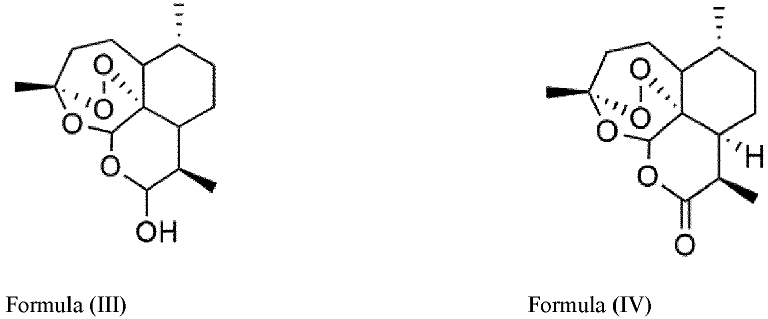 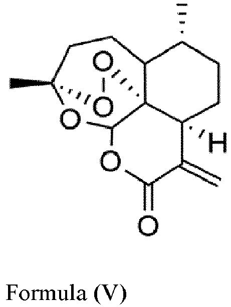
and, where applicable, pharmaceutically acceptable salts of.the aforementioned compounds of formulae (I) and (II) wherein in
formula (I)
the arrow denotes the bond between the depicted oxygen atom to the residue R¹
n is an integer of more than 1, preferably 2 to 10, more preferably 2, 3 or 4 and even more preferably 2 or 3
R¹ is a radical that is n times substitued by the residue depicted in the rounded bracket, and is preferably C₁-C₁₈-alkyl or C₂-C₁₈-alkenyl or -(CO)ₙ(R³), wherein the carboyl groups together with the oxygen bound to the residue R¹ form a carboxylic ester moiety and R³ is C₁-C₁₈-alkane-n-yl or C₂-C₁₈-alkene-n-yl
whereby
the aforementioned C₁-C₁₈-alkyl, C₂-C₁₈-alkenyl, C₁-C₁₈-alkane-n-yl, C₂-C₁₈-alkene-n-yl groups are
• either not, once, twice or more than twice interrupted by non-successive functional groups selected from the group consisting of:
-O-, -S-, -SO₂-, -SO-, -SO₂NR⁴-, NR⁴SO₂-, -NR⁴-, -CO-, -O(CO)-, (CO)O-,-O(CO)O-, -NR⁴(CO)NR⁴-, NR⁴(CO)-, -(CO)NR⁴-, -NR⁴(CO)O-,-O(CO)NR⁴-,
and
• either not, additionally or alternatively either once, twice or more than twice interrupted by bivalent residues selected from the group consisting of heterocyclo-diyl, and aryldiyl,
and
• either not, additionally or alternatively either once, twice or more than twice substituted by substituents selected from the group consisting of:
hydroxy, halogen, cyano, azido, C₆-C₁₄-aryl, C₁-C₈-alkoxy, C₁-C₈-alkylthio,-SO₃M, -COOM, PO₃M₂, -PO(N(R⁵)₂)₂, PO(OR⁵)₂, -SO₂N(R⁴)₂, -N(R⁴)₂,-CO₂N(R⁵)₂, -COR⁴, -OCOR⁴, -NR⁴(CO)R⁵, -(CO)OR⁴, -NR⁴(CO)N(R⁴)_{2.}
and whereby in formula (II)
R² is C₁-C₁₈-alkyl or C₂-C₁₈-alkenyl or -(CO)R³, wherein the carboyl groups together with the oxygen bound to the residue R1 form a carboxylic ester moiety and R³ is C₁-C₁₈-alkyl or C₂-C₁₈-alkenyl whereby
the aforementioned C₁-C₁₈-alkyl and C₂-C₁₈-alkenyl groups are
• either not, once, twice or more than twice interrupted by non-successive functional groups selected from the group consisting of:
-O-, -S-, -SO₂-, -SO-, -SO₂NR⁴- , NR⁴SO₂-, -NR⁴-, -CO-, -O(CO)-, (CO)O-,-O(CO)O-, -NR⁴(CO)NR⁴-, NR⁴(CO)-, -(CO)NR⁴-, -NR⁴(CO)O- or -O(CO)NR⁴-
and are
• either not, additionally or alternatively either once, twice or more than twice interrupted by bivalent residues selected from the group consisting of heterocyclo-diyl, and aryldiyl,
and are
• either not, additionally or alternatively either once, twice or more than twice substituted by substituents selected from the group consisting of:
hydroxy, halogen, cyano, azido, C₆-C₁₄-aryl, C₁-C₈-alkoxy, C₁-C₈-alkylthio,-SO₃M, -COOM, PO₃M₂, -PO(N(R⁵)₂)₂, PO(OR⁵)₂ , -SO₂N(R⁴)₂, -N(R⁴)₂,-CO₂N(R⁵)₂, -COR⁴, -OCOR⁴, -NR⁴(CO)R⁵, -(CO)OR⁴ or -NR⁴(CO)N(R⁴)₂
whereby in all formulae above where used
R⁴ is independently selected from the group consisting of hydrogen, C₁-C₈-alkyl, C₆-C₁₄-aryl, and heterocyclyl or N(R⁴)₂ as a whole is a N-containing heterocycle,
R⁵ is independently selected from the group consisting of C₁-C₈-alkyl, C₆-C₁₄-aryl, and heterocyclyl or N(R⁵)₂ as a whole is a N-containing heterocycle and
M is hydrogen, or 1/q equivalent of an q-valent metal ion or is an ammonium ion or a guanidinium ion or a primary, secondary, tertiary or quarternary organic ammonium ion, in particular those of formula [N(C₁-C₁₈-alkyl)ₛHₜ]⁺ wherein s is 1,2,3 or 4 and t is (4-s).
and wherein
the compound of formula (III) is dihydroartemisin, the compound of formula (IV) is artemisinin and the compound of formula (V) is artemisitene.

3. The compounds for use in the treatment of COVID-19 according to claim 1 or 2 wherein the compounds of formula (II) are those of formula (IIa), artemether, and of formula (IIb), artesunate, and pharmaceutically acceptable salts of artesunate.

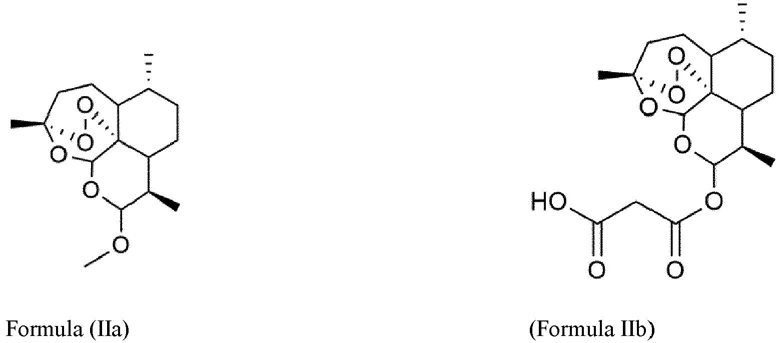

4. Antiviral composition for use in the treatment of COVID-19 comprising one or more than one antiviral compound comprising at least one 1,2,4-trioxane moiety and preferably one or more than one antiviral compound selected from those of formulae (I) to (V) according to claim 2.

5. The antiviral composition for use in the treatment of COVID-19 according to claim 4 further comprising at least one compound, for example one, two, three, four, five, six, seven, eight, nine, ten or all compounds selected from the group consisting of those of formulae (VI) to (XVI) 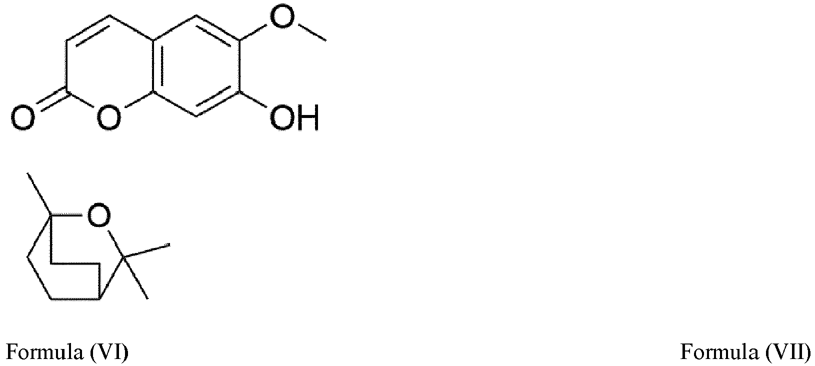 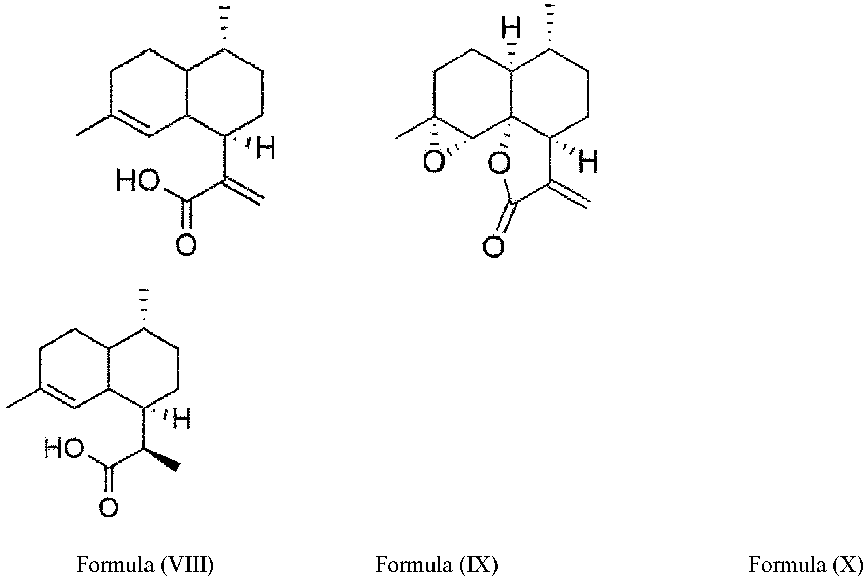 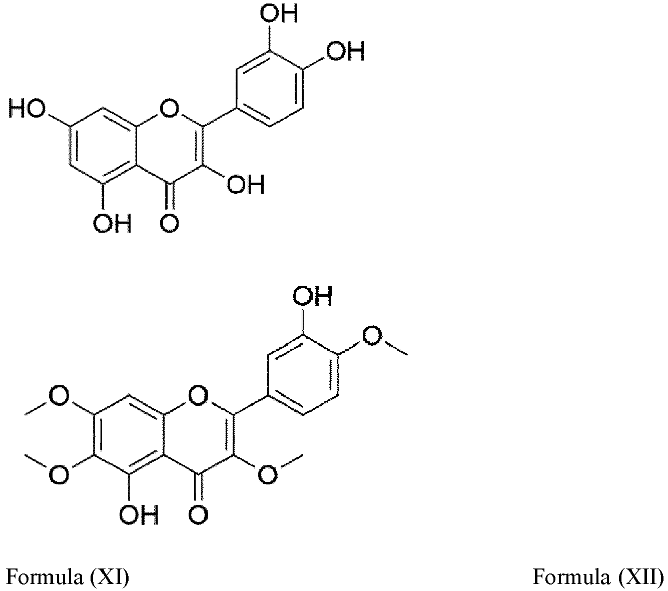 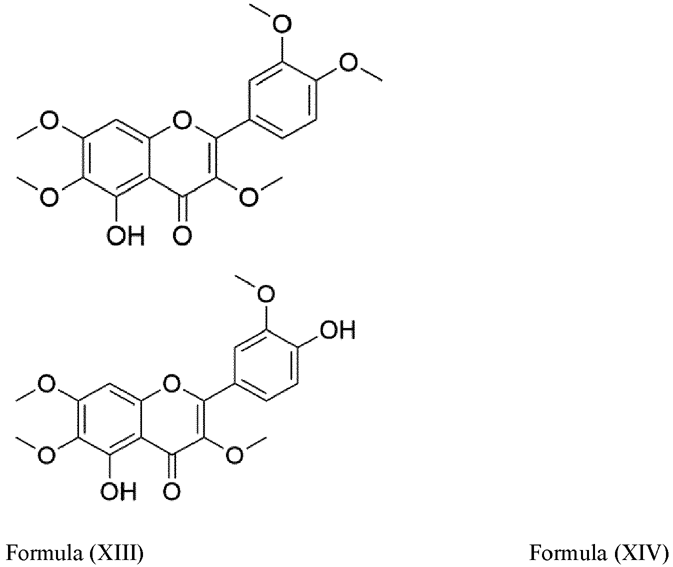 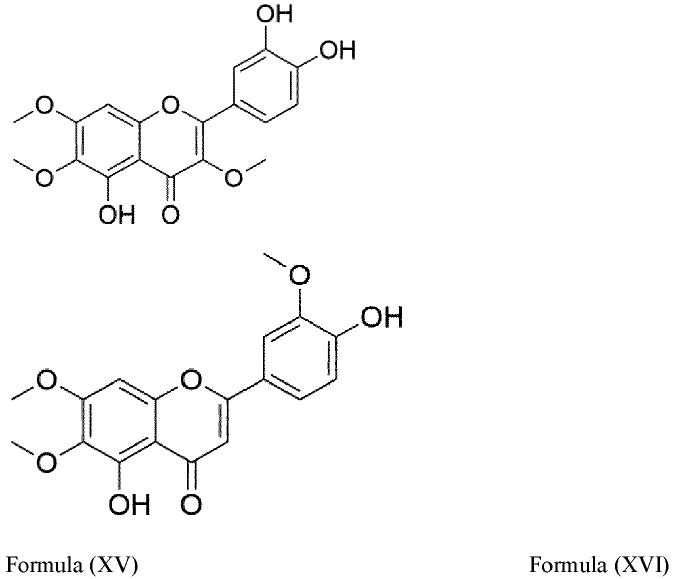 whereby the compound of formula (VI) is scopoletin, the compound of formula (VII) is 1,8-cineole, the compound of formula (VIII) is artemisinic acid., the compound of formula (IX) is arteannuin-B, the compound of formula (X) is dihydroartemisinic acid, the compound of formula (XI) is fisetin, the compound of formula (XII) is casticin, the compound of formula (XII) is artemetin, the compound of formula (XIV) is chrysoplenetin, the compound of formula (XV) is chrysoplenol-D and the compound of formula (XVI) is cirsilineol.

6. A pharmaceutical composition for use in the treatement of COVID-19 comprising an antiviral compound according to anyone of claims 1 to 3 or an antiviral composition of claims 4 or 5.

7. An antiviral composition according to claim 4 or 5 or a pharmaceutical composition according to claim 6 comprising the plant *Artemisia annua* or parts thereof or extracts obtained by extraction of the plant *Artemisia annua* or parts thereof.

8. The antiviral composition according to claim 4 or 5 or the pharmaceutical composition according to claim 7 wherein the *Artemisia annua* plant is the Apollon variety.

9. The pharmaceutical composition of any one of claims 6 to 8 further comprising at least one additional agent selected from: a virus entry inhibitor, a virus early transcription event inhibitor, another virus RNA polymerase inhibitor, a virus protease inhibitor, a virus terminase inhibitor, a virus maturation inhibitor, an inhibitor of another target in the virus life cycle and a vaccine.

10. The pharmaceutical composition of any one of claims 6 to 8 further comprising chloroquine, hydroxychloroquine or a pharmaceutically acceptle salt of the aforementioned and / or remdesivir and/or lopinavir and/or ritonavir and/or triazavirin.

11. A method of inhibiting the replication of SARS-CoV-2 comprising exposing the virus to an effective amount of the antiviral compound or antiviral composition or pharmaceutical compositions according to any one of claims 1 to 10.
